# EUROPEAN PATENT SPECIFICATION

(11) **EP 1 628 640 B1**
(45) Date of publication and mention of the grant of the patent: **14.11.2007**
(21) Application number: 04741678.9
(22) Date of filing: 28.05.2004
(51) Int. Cl.: A61K 9/16

(54) **MICROCAPSULES BY COACERVATION CONTAINING A PHARMACEUTICAL INCORPORATED IN THE ETHYLCELLULOSE COATING**
DURCH COAZERVATION HERGESTELLTE MIKROKAPSELN MIT EINEM WIRKSTOFF IN ETHYLCELLULOSE ALS HÜLLPOLYMER
MICROCAPSULES PRODUITES PAR COACERVATION CONTENANT UN AGENT PHARMACEUTIQUE INCORPORE DANS LE REVETEMENT D'ETHYLCELLULOSE

(30) Priority: 30.05.2003 IT MI20030109
(43) Date of publication of application: 01.03.2006
(73) Proprietor: Eurand S.P.A., 20060 Pessano Con Bornago (IT)
(72) Inventor: GOLZI, Roberto, I-26100 Cremona (IT); BOLTRI, Luigi, I-20041 Agrate Brianza (IT); STOLLBERG, Christian, I-20063 Cernusco Sul Naviglio (IT)
(74) Representative: Gervasi, Gemma
(86) International application number: PCT/EP2004/050962
(87) International publication number: WO 2004/105725

(56) References cited:
- EP-A- 0 475 536
- EP-A- 0 706 794
- WO-A-98/00116
- DE-A- 19 930 795
- FR-A- 2 758 461
- US-A- 5 252 337
- US-A1- 2002 064 563
- DATABASE WPI Section Ch, Week 198730 Derwent Publications Ltd., London, GB; Class A96, AN 1987-209277 XP002267560 & JP 62 135419 A (IKEDA MOHANDO) 18 June 1987 (1987-06-18)

## Description

### Field of the invention

The present invention relates to the field of microcapsules.

### Prior art

The coating of inert cores with active ingredients is a well known method in the pharmaceutical field and it is generally carried out using coating pans or fluid beds. The coating may be applied through a powder layering process or a solution layering process. In both techniques the application of the active principle to the surface of inert beads is carried out by means of a binder.

This type of formulation in microcapsules is generally used in order to increase the exposure of the active principle to the aqueous medium and thus to increase the bioavailability of poorly soluble drugs.

Also known is the art is the possibility to modulate the release of this type of formulations by covering the layer of active principle with a further film (US 6,077,533) containing a polymer that controls release, masks the unpleasant taste or, yet again, isolates the active Ingredient from direct exposure to light or air.

Furthermore, in US 4,261,971 it is disclosed a gastro-resistant formulation obtained by coating the surface of a core of inert material by a solution layering process, wherein a solution comprising an active principle and a gastro-resistant polymer is sprayed on the inert cores in a fluid bed apparatus.

A well known technique used for the preparation of microcapsules is the coating of crystals or granules containing active substances by coacervation by phase separation in order to provide them with taste masking or modified release properties (U.S. Pat. No. 4,411,933; U.S. Pat. No. 3,860,733). This process may take place both in an aqueous environment and in an organic solvent. In the first case, the polymer is separated from the solution by modifying its solubility either by means of variations in the pH and/or temperature or by adding phase-separation inducing agents to the solutions; in the second case separation of the polymer is obtained by a variation in solubility caused by variation in the temperature of the solution in which the polymer is dissolved.

The above described powder layering processes performed by applying the active ingredient with fluid bed apparatus or coating pans are time consuming and are relatively costly multi-step processes. Often the therapeutic dose of the pharmaceutical is very small, for example from 1 to 10 mg: the production of granules or pellets with a small active ingredient content could create problems of content uniformity. Moreover, loading the active ingredient on cores by powder layering, in the case of low dosage substances, requires the formulation of mixtures of powders with extremely low active ingredient concentrations to guarantee homogeneous distribution on the substrate; this requires the use of large volumes of diluents and very long coating times, which is reflected in high process costs. If on the other hand microcapsules containing only the active ingredient were to be produced problems of dosage would still be created due to the need for a high level of dilution of the microcapsules in the final pharmaceutical form.

Therefore, new procedures with improved efficiency are still required to deposit pharmaceutical particles on cores, in particular a procedure with high repeatability and low industrial cost that guarantees uniform deposition of pharmaceutical particles on the surface of cores, even when working with very small-doses of pharmaceutical.

### Summary of the Invention

It has now surprisingly been found that, using the technique of microencapsulation by coacervation by means of phase separation, it is possible to produce microcapsules with a core consisting for example of a granule, a non-pareil, a pellet of excipient, usually of inert material, which is coated with ethylcellulose wherein the active ingredient is dispersed in the form of solid particles.

The microcapsules thus produced may advantageously carry the active substances in taste-masking and/or modified release formulations.

The procedure to obtain the aforesaid microcapsules consists essentially in producing a homogeneous solution of ethylcellulose in a suitable solvent in which the cores to be coated, the particles of active ingredient and, optionally, membrane additives are dispersed in suspension. Per se known methods are then used to cause insolubilisation of the polymer, which gels (coacervates) around the cores to form the microcapsules. For example, phase separation may be performed through variation in temperature or in pH or by adding phase-separation inducing substances that cause insolubilisation of the polymer. Finally, the microcapsules obtained are subjected to hardening, if required, and recovered.

The fact that the solution subjected to coacervation not only contains the customary membrane agents, but also the pharmaceutical particles which, following coacervation, are incorporated into the coating layer of the cores, is characteristic of the present invention.

The solvent utilised in the coacervation procedure is chosen so as to dissolve the coating polymer but not the active ingredient(s), the membrane additive(s) and the inert cores.

In the case of coating polymers insoluble in water, the coacervation solvent is an organic solvent, such as cyclohexane, or a mixture of organic solvents; in the case of coating polymers soluble in aqueous medium, the coacervation solvent is preferably water with the addition of buffer salts if required.

Among the additives that may be used in an organic solvent environment we may cite lactose, mannitol or water-soluble polymers such as polyvinylpyrrolidone and its derivates or cellulose derivates such as HPMC, MC, HPC. Other additives to use in an organic environment that may be included in the membrane together with the active ingredient may be fast swelling ones such as sodium carboxylmethylamide (Explotab), croscarmellose (AcDiSol), Crospovidone, pregelatinized starch (Starch 1500). Also included in the membrane may be pH modifiers (organic acids, bases, salts, buffer systems) with the aim of facilitating complete dissolution and diffusion of the active ingredient.

Among the excipients that can be used in an aqueous solvent environment we can cite calcium and magnesium salts such as dibasic calcium phosphate, calcium sulphate, barium sulphate, calcium carbonate, magnesium carbonate and silicates.

Phase separation is performed with per se known means, for example through variation in temperature or in pH, or by adding phase-separation inducing substances that cause insolubilization of the polymer. The microcapsules obtained are then subjected to hardening, if required, and finally recovered.

### Description of the figures

Figure 1 shows a microscope image of a microcapsule obtained in accordance with comparative Example 3.
Figure 2 shows a microscope image of a microcapsule obtained in accordance with Example 11 of the present invention.
Figure 3 shows a microscope image of a microcapsule obtained in accordance with Example 8 of the present invention.

### Detailed description of the Invention

The present invention relates to a method of preparing microcapsules containing at least one active ingredient comprising the application of ethylcellulose membrane containing at least one active ingredient and, optionally, at least one membrane additive to a core having dimensions ranging from 50 to 1200 µm wherein said application is carried out by the process of coacervation by means of phase separation.

The microcapsules obtained with the process of the invention are characterised by the dispersion of the active ingredient(s)'and, if required, membrane additive(s) in the polymeric membrane in the form of solid particles.

Microencapsulation by phase separation is a per se known procedure. The present invention is characterised by the fact that the coacervation solution contains, in addition to the customary membrane agents, also active ingredient particles, suspended therein, which, following coacervation, are incorporated in the coating membrane of the cores.

More specifically, the procedure according to the invention comprises the following steps:
(a) forming a solution of ethylcellulose in an aqueous or in organic solvent;
(b) suspending the cores, the particles of active ingredient and, optionally, any membrane additives in the solution obtained in (a),
(c) causing coacervation of ethylcellulose in the suspension obtained in (b) by means of phase separation, thus obtaining cores coated with a polymeric membrane incorporating the active ingredient,
(d) if necessary, subjecting the microcapsules to a hardening treatment of membrane,
(e) preferably washing and recovering the microcapsules thereby obtained.

In step (a) the solvent or mixtures of solvents used to form the solution must be able to dissolve the polymer but not the active ingredient and the membrane additives, which remain dispersed in the form of solid particles in suspension. A particularly preferred solvent to be used in step a) is cyclohexane.

In step (b), the cores, the active ingredient and any membrane additive are added to the polymer solution under stirring; the order in which the different components are added is not determining.

Step a) and step b) may be also carried out as a single step, that is by addition of the polymer, the cores, the active ingredient and any membrane additive to solvent under stirring.

According to the present invention with the term "active ingredient" is meant any active ingredient with biological activity, in particular pharmaceuticals, also including mixtures of two or more of these. Non limiting examples of pharmaceuticals that may be used according to the present invention are: bronchodilating agents, CNS stimulants, antidepressants, anti-inflammatories, antispasmodics and antiulceratives.

The membrane additives have solubility characteristics opposed to those of the membrane: In the case of water-soluble membranes, additives that are insoluble in water are used; in the case of membranes insoluble in water, water-soluble additives are used. Examples of preferred water soluble additives are lactose, mannitol, polyvinylpyrrolidone, hydroxypropylmethylcellulose, methylcellulose, hydroxypropylcellulose, swelling agents such as carboxymethylamide, croscarmellose, crospovidone, pregelatinized starch. Other examples may be pH modifiers such as dibasic sodium phosphate, citric acid, tartaric acid, fumaric acid, potassium hydrogen phosphate; once in contact with the dissolution means, these produce a pH (ranging from 1 to 9, more preferably from 2 to 7.5). Among the additives insoluble in water we may cite calcium and magnesium salts such as dibasic calcium phosphate, calcium sulphate, barium sulphate, calcium carbonate, magnesium carbonate, silicates.

In step (c), phase separation can take place by means of various techniques, per se known, such as temperature or pH variation or by adding substances, said phase-separation inducing agents, that modify the solubility equilibrium characteristics of the coating polymer dissolved and thus cause its insolubilisation. The technique used to obtain phase separation varies depending on the solvent in which the micro-encapsulation is carried out.

More specifically, in case the micro-encapsulation is carried out in an organic solvent the active ingredient, previously dispersed together with the cores and with any membrane additives, is subjected to cooling. During the cooling phase, through the effect of separation of the polymer the active ingredient remains incorporated in the form of solid particles into the gelified layer of the polymer together with any additives added in a mixture with it.

In the case of microencapsulation in an aqueous environment, after having suitably dissolved the polymer, for example CAP in a phosphate buffer, the cores are dispersed and the active ingredient is added under stirring at a constant speed. In this case phase separation is obtained by means of variations in the pH and/or temperature or by adding phase-separation inducing agents to the solutions. According to a preferred embodiment of the present invention phase separation is performed by progressively adding a phase-separation inducing agent, for example a saturated solution of sodium sulphate, observing the solution separating and becoming viscous, an indication that the polymer has separated.

In the optional step (d) the microcapsules are subjected to a hardening treatment of the membrane; these treatments are per se known and produced for example, in case of microencapsulation in an aqueous environment by adding citric acid while in case of encapsulation in an organic environment by cooling to ambient temperature.

The amount of polymer, its nature and any additives introduce contribute towards regulating release of the active ingredient making it possible to modulate the degree of taste masking and/or modified release.

In step (e) the microcapsules are preferably washed to eliminate any excess residues of reagent, and after suitable decantation are recovered for example by filtration and then dried.

If necessary, one or more further protective coating layers may be applied on the microcapsules of the invention.

The present invention also relates to new microcapsules obtainable by the above described process. The microcapsules of the invention comprise cores coated with a polymer membrane wherein an active ingredient and, if required, membrane additives are dispersed in the form of solid particles

The core preferably constitutes 50-95%, or more preferably 60-70% by weight of the microcapsule. It has a dimension ranging from 50 to 1200 µm, preferably from 100 to 500 µm.

The main function of the core is to provide a uniform and reproducible substrate suitable for microencapsulation; any core that satisfies the aforesaid dimensional requirements may be used in the present invention (for example, microgranules, granules, pellets, etc.); preferred cores are non-pareil seeds, which, by means of the essentially regular spherical shape, allow uniform encapsulation with the coating polymer.

The core is essentially constituted by inert excipients, also mixed with one another, for example sucrose, lactose, microcrystalline cellulose, starch, talc, gum arabic, glyceryl monostereate, glyceryl behenate, etc.. The cores preferably have no active ingredients; nonetheless, in some case it is also possible to use cores inside which an active ingredient is dispersed; this is useful for example when wishing to load high amounts of active principle and/or modulate release over a longer period of time; in these cases the microcapsule will not only contain the active ingredient in the wall of the coating, but also inside the core.

The polymer that constitutes the coating membrane represents in general from 2 to 40% by weight of the microcapsule, preferably from 2 to 20%.

The particles of active ingredient contained in the membrane have a smaller mean diameter than the diameter of the core and are dispersed in the polymer membrane that coats the microcapsule in the form of solid particles.

The active ingredient particles preferably have dimensions ranging from 0.1 to 80 µm, more preferably from 1 to 30 µm; the active ingredient is contained in the microcapsules in a percentage by weight preferably ranging from 0.1 to 40%, more preferably from 0.2 to 21 % by weight of the microcapsule.

The present inventors have surprisingly found that the coacervation process of the invention allows to obtain microcapsules that present a different distribution of the active principle in the membrane layer depending on the type of polymer utilised. Furthermore, the present inventors have surprisingly found that, by modulating the type and amount of polymer used in the process of the invention, it is possible to obtain microcapsules wherein the taste of the active principle is masked and/or its release modified without the need for further coating layers.

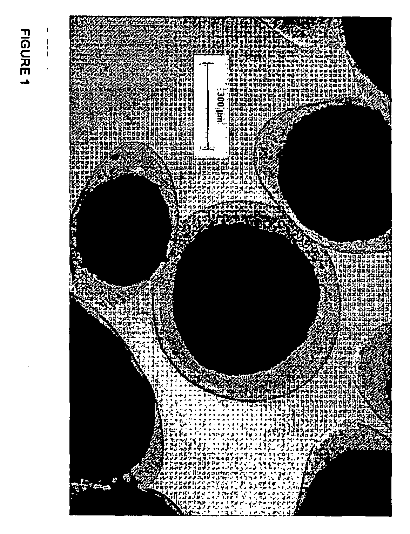

In the case of microencapsulation with a water-soluble polymer, the microcapsules obtained with the process of the invention are characterised by the fact that the active ingredient particles are prevalently disposed at the interface between the polymer and the core. In fact, as can be seen in Figure 1 (comparative example 3), in this case the particles of active principle are distributed within the polymeric wall with a concentration that decreases progressively moving from the core towards the distal part of the membrane that may become essentially null at the level of the external surface.

This type of microcapsules have the advantage that they may be used for obtaining a taste masking effect, due to the absence of active principle in the distal part of the membrane and a modified release without the need for further protective layers. The modified release, in particular delayed release, which is attained with the polymers used in the present invention, depends on the amount and type and of polymer. The same polymers are also effective in taste masking application.

It is also important to stress that the aforesaid separation of the pharmaceutical inside the membrane does not influence the homogeneity of distribution of the pharmaceutical on the cores; in fact, the deposition percentage of the active ingredient on each surface unit of the core remains essentially constant in the entire batch subjected to microencapsulation, thus guaranteeing the necessary uniformity of dosage on the surface of the cores.

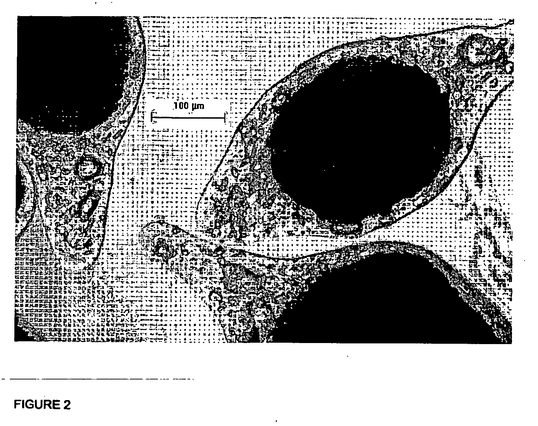

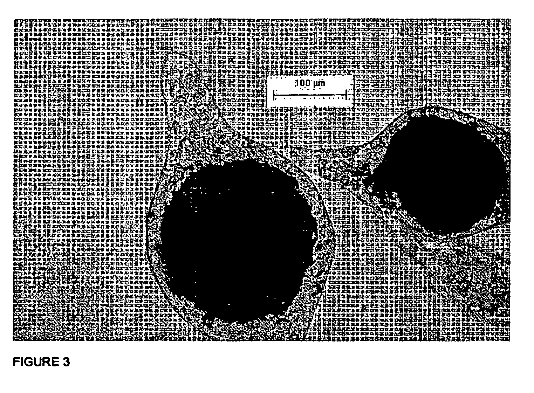

In case the microencapsulation is carried out using a water insoluble polymer microcapsules are obtained wherein the particles of active principle are homogeneously dispersed within the coating membrane (Figures 2 and 3).

As demonstrated in the Examples below, in this case it is possible to obtain microcapsules with a modified release or/and taste masking. Both the amount of polymers and the presence of additives influence the dissolution rate of the active principle, thus attaining the desired modified release and/or taste masking.

Besides the pharmaceutical and the coating polymer, the membrane may also contain membrane additives useful to modulate the characteristics, for example permeability, mechanical resistance, plasticity, or to correct the organoleptic aspects (colour, odour, taste). The membrane additives have solubility characteristics opposed to those of the membrane: in the case of water soluble membranes, additives that are insoluble in water are used; in the case of membranes insoluble in water, water soluble additives are used. Examples of water soluble additives are lactose, mannitol, polyvinylpyrrolidone, hydroxypropylmethylcellulose, methylcellulose, hydroxypropylcellulose, swelling agents such as carboxymethylamide, croscarmellose, crospovidone, pregelatinized starch. Other examples may be pH modifiers such as dibasic sodium phosphate, citric acid, tartaric acid, fumaric acid, potassium hydrogen phosphate; once in contact with the dissolution means, these produce a pH (ranging from 1 to 9, more preferably from 2 to 7.5). Among the additives insoluble in water we may cite calcium and magnesium salts such as dibasic calcium phosphate, calcium sulphate, barium sulphate, calcium carbonate, magnesium carbonate and silicates.

Some of the above additives, for example, polyvinylpirrolidone may also affect the dissolution rate of the active ingredient.

The membrane additives have a smaller mean diameter than the diameter of the cores, preferably ranging from 0.1 to 80 µm, more preferably from 7 to 30 µm, and preferably constitute from 2 to 20% by weight, more preferably from 3 to 10 % by weight of the microcapsule.

A further coating can be performed to further modulate the release of the active ingredient. The material used for the coating layers and the process for their application are those well known to the experts of the field.

With the instant invention, deposition of reproducible quantities of active ingredient on the surface of pharmaceutical cores has thus been attained using an easy to produce process; this procedure has proved to be reliable even when the pharmaceutical to be incorporated is present in low doses; moreover, microcapsules may be obtained wherein the taste of the active principle has been masked and/or its release modified without the necessity of applying further coating layers.

The present invention is now illustrated by means of the following examples.

### EXPERIMENTAL PART

### Example 1

At 80°C dissolve the ethylcellulose (10 parts) and polyethylene (20g) in cyclohexane (1000 parts) and disperse therein the MCC cores (Celphere FMC) (184 parts). Under stirring add ground Caffeine (D 4.3 = 25 µm) (4 parts), polyvinylpyrrolidone (2 parts). Again under stirring cool slowly to ambient temperature. The microcapsules obtained are separated by decantation, filtered and dried.

### Example 2

Dissolve ethylcellulose (12 parts) and polyethylene (20g) in cyclohexane (1000 parts) at 80°C and disperse therein the MCC cores (Cellets PHARMATRANS) (172 parts), under stirring add Metoclopramide hydrochloride (D 4.3 = 30µm) (12 parts) and mannitol (4 parts). Again under stirring cool to ambient temperature. The microcapsules obtained are separated, filtered and dried.

### Example 3 (comparative example)

Dissolve the cellulose acetate phthalate (CAP) (140 parts) In water (6000 parts) containing a buffer mixture constituted by sodium and /or potassium phosphates and create a pH ranging from 6.0 to 6.5 with ionic strength of 0.5.

Disperse the cores, 700 parts, constituted by granules or pellets, based on insoluble inorganic salts and binding substances such as triglycerides of fatty organic acids, obtained by granulation; add under stirring Ibuprofen, (160 parts) of dimensions ranging from 10 to 30 µm.

Add the phase-separation inducing agent, constituted by a 20% solution of sodium sulphate to promote phase separation of the CAP, then add a solution of citric acid to cause hardening of the membrane. Separate by filtering and dry the microcapsules obtained.

The microcapsules obtained, when analysed with an electronic microscope (Fig 1), show a distribution of the particles of the active principle within the polymeric membrane in the form of solid particles that are prevalently disposed at the interface between the polymer and the core.

### Example 4

Dissolve the ethylcellulose 100STD p (30g) and polyethylene (60g) in cyclohexane (3000 g) at high temperature and disperse therein the inert cores (273g) (size 350 µm); under stirring add anhydrous micronised theophylline (60g). Again under stirring cool slowly to ambient temperature. The microcapsules obtained are separated by decantation, washed, filtered, and dried.

A dissolution test is carried out on the microcapsules thus obtained comparing them with anhydrous theophylline in crystalline form.

| //// | Dissolution % at pH 6.8 | | | |
|---|---|---|---|---|
| Assay(mg active principle/g microcapsule) | 5 min | 15 min | 30 min | 60 min |
| 160 mg/g | 18 | 41 | 60 | 83 |
| Anhydrous Theophylline 160 mg | 65 | 100 | // | // |

The results obtained In the dissolution test demonstrate that a modified release was obtained with the microencapsulation. The dissolution test was performed according to USP 26.

### Example 5

Dissolve the ethylcellulose 100STD p (30g) and polyethylene (60g) in cyclohexane (3000 g) at high temperature and disperse therein the inert cores (273g) (size 500 µm), under stirring add anhydrous micronised theophylline (60g). Again under stirring cool slowly to ambient temperature. The microcapsules obtained are separated by decantation, washed, filtered, and dried.

### Example 6

Dissolve the ethylcellulose 100STD p (8g) and polyethylene (20g) in cyclohexane (1000 g) at high temperature and disperse therein the inert cores (92g) (size 200 µm), under stirring add anhydrous micronised theophylline (17g) and polyvinylpyrrolidone CL (3g). Again under stirring cool slowly to ambient temperature. The microcapsules obtained are separated by decantation, washed, filtered, and dried.

### Example 7

A dissolution test is carried out on the microcapsules obtained in Examples 5 and 6 according to the method previously described for Example 4

| example | //// | Dissolution % at pH 6.8 | | | |
|---|---|---|---|---|---|
| | Assay | 5 min | 15 min | 30 min | 60 min |
| 6 | 159 mg/g | 18 | 37 | 55 | 75 |
| | Assay | 10 min | 20 min | 30 min | 60 min |
| 7 | 142 mg /g | 28 | 64 | 84 | 94 |
| | Anhydrous theophylline 160 mg | 100 | // | // | // |

The dissolution test demonstrate that by introducing an additive (Ex. 7) it is possible to further modify the release.

### Example 8

Dissolve the ethylcellulose 100STD p (22g) and polyethylene (60g) in cyclohexane (3000 g) at high temperature and disperse therein the inert cores (273g) (size 350 µm), under stirring add caffeine (60g). Again under stirring cool slowly to ambient temperature. The microcapsules obtained are separated by decantation, washed, filtered, and dried.

The microcapsules obtained, when analysed with an electronic microscope (Fig 3), show a distribution of the particles of the active principle within the polymeric membrane in the form of solid particles that are prevalently disposed at the interface between the polymer and the core. A dissolution test is carried out on the microcapsules thus obtained compared to caffeine raw material showing the modified release obtained.

| //// | Dissolution % at pH 1.2 | | | |
|---|---|---|---|---|
| Assay | 60 min | 120 min | 240 min | 480 min |
| 169 mg/g | 47 | 57 | 68 | 71 |
| Caffeine 170 mg | 100 | | | |

| | | | | |
|---|---|---|---|---|
| Dissolution test was performed according to USP 26. | | | | |

### Example 9

Dissolve the ethylcellulose 100STD p (8g) and polyethylene (20g) in cyclohexane (1000 g) at high temperature and disperse therein the inert cores (92g) (size 100 µm), under stirring add omeprazole (17g) and polyvinylpyrrolidone CL (3g). Again under stirring cool slowly to ambient temperature. The microcapsules obtained are separated by decantation, washed, filtered, and dried.

### Example 10

A dissolution test is carried out on the microcapsules obtained in Example 9 showing the influence of the particle size of the inert core on the modified release.

| | | Dissolution % at pH 6,8 | | | |
|---|---|---|---|---|---|
| example | Assay | 30 min | 60 min | 120 min | 240 min |
| 9 | 190 mg/g | 13 | 20 | 27.5 | 38 |

| | | | | | |
|---|---|---|---|---|---|
| Dissolution test was performed according to USP 26 | | | | | |

### Example 11

Dissolve the ethylcellulose 100STD p (5g) and polyethylene (20g) in cyclohexane (1000 g) at high temperature and disperse therein the inert cores (77g) (size 200 µm), under stirring add fluoxetine (6g) and polyvinylpyrrolidone CL (3g). Again under stirring cool slowly to ambient temperature. The microcapsules obtained are separated by decantation, washed, filtered, and dried.

The microcapsules obtained, when analysed with an electronic microscope (Fig 2), show a homogeneous distribution of the particles of the active principle within the polymeric membrane in the form of solid particles.

A dissolution test is carried out on the microcapsules thus obtained compared to fluoxetine raw material.

The very low amount d solved after 5 min indicates the achievement of a taste masking effect due to the coating polymer.

| | | Dissolution % at pH 6,8 | | | | |
|---|---|---|---|---|---|---|
| Example | Assay | 5 min | 10 min | 20 min | 30 min | 60 min |
| 11 | 67 mg/g | 2 | 76 | 85 | 90 | 95 |
| Fluoxetine | 60 mg | 55 | 75 | 100 | | |

| | | | | | | |
|---|---|---|---|---|---|---|
| Dissolution test was performed according to USP 26. | | | | | | |

## Claims

1. Method of preparing microcapsules containing at least one active ingredient comprising the application of a ethylcellulose membrane containing at least one active ingredients and, optionally, at least one membrane additive to a core having dimensions ranging from 50 to 1200 µm wherein said application is carried our by the process of coacervation by means of phase separation.

2. Method as claimed in claim 1 comprising the following steps:
(a) forming a solution of ethylcellulose in an aqueous or in organic solvent;
(b) suspending the cores, the particles of active ingredient and, optionally, any membrane additive in the solution obtained in (a),
(c) causing coacervation of ethylcellulose in the suspension obtained in (b) by means of phase separation,
(d) optionally, subjecting the microcapsules to a hardening treatment of the membrane.
(e) recovering the microcapsules thereby obtained.

3. Method as claimed in claim 2 wherein step a) and b) are carried out as a single step.

4. Method as claimed in claim 2 or 3 wherein the solvent used in step a) is cyclohexane.

5. Method as claimed in claim 2 to 4 wherein the additive added in step b) is selected from lactose, mannitol, polyvinylpyrrolidone, hydroxypropylmethylcellulose, methylcellulose, hydroxypropylcellulose, swelling agents such as sodium carboxymethylamide, croscarmellose, crospovidone, pregelatinized starch and pH modifiers

6. Method as claimed in claims 2 to 5, wherein in step c) phase separation takes place by variation in temperature.

## Patentansprüche

1. Verfahren zur Herstellung von Mikrokapseln, enthaltend mindestens einen Wirkstoff, umfassend die Aufbringung einer Ethylcellulose-Membran, enthaltend mindestens einen Wirkstoff und, gegebenenfalls, mindestens einen Membranzusatzstoff auf einen Kern mit Abmessungen in einem Bereich von 50 bis 1200 µm, wobei genannte Aufbringung durch das Verfahren der Koazervierung mittels Phasentrennung ausgeführt wird.

2. Verfahren gemäß Anspruch 1, umfassend die folgenden Schritte:
(a) Bilden einer Lösung des Ethylcellulose-Polymers in einem wässrigen oder in organischem Lösungsmittel;
(b) Suspendieren der Kerne, der Partikel des Wirkstoffs, und gegebenenfalls, eines beliebigen Membranzusatzstoffes in der in (a) erhaltenen Lösung;
(c) Verursachen einer Koazervierung des Ethylcellulose-Polymers in der in (b) erhaltenen Suspension mittels Phasentrennung,
(d) gegebenenfalls, Unterziehen der Mikrokapseln einer Härtungsbehandlung der Membran,
(e) Rückgewinnung der **dadurch** erhaltenen Mikrokapseln.

3. Verfahren gemäß Anspruch 2, wobei Schritt a) und b) als ein einziger Schritt ausgeführt werden.

4. Verfahren gemäß Anspruch 2 oder 3, wobei das in Schritt a) verwendete Lösungsmittel Cyclohexan ist.

5. Verfahren gemäß den Ansprüchen 2 bis 4, wobei der in Schritt b) zugegebene Zusatzstoff ausgewählt wird aus Lactose, Mannit, Polyvinylpyrrolidon, Hydroxypropylmethylcellulose, Methylcellulose, Hydroxypropylcellulose, Quellmitteln wie z. B. Natrium-Carboxymethylamid, Croscarmellose, Crospovidon, vorgelatinisierter Stärke und pH-Wert-Modifizierungsmitteln.

6. Verfahren gemäß den Ansprüchen 2 bis 5, wobei in Schritt c) die Phasentrennung durch Variieren der Temperatur stattfindet.

## Revendications

1. Procédé de préparation de microcapsules contenant au moins un principe actif, comprenant l'application d'une membrane d'éthylcellulose contenant au moins un principe actif et, éventuellement, au moins un additif de membrane sur une partie centrale dont les dimensions vont de 50 à 1200 µm, ladite application étant réalisée par le processus de coacervation au moyen d'une séparation de phases.

2. Procédé selon la revendication 1, comprenant les étapes suivantes qui consistent à:
(a) former une solution d'éthylcellulose dans un solvant aqueux ou dans un solvant organique;
(b) mettre les parties centrales, les particules du principe actif et, éventuellement, tout additif de membrane, en suspension dans la solution obtenue en (a),
(c) provoquer la coacervation de l'éthylcellulose dans la suspension obtenue en (b) au moyen d'une séparation de phases,
(d) éventuellement, soumettre les microcapsules à un traitement de durcissement de la membrane,
(e) récupérer les microcapsules obtenues de cette façon.

3. Procédé selon la revendication 2, dans lequel l'étape a) et l'étape b) sont réalisées sous la forme d'une seule étape.

4. Procédé selon la revendication 2 ou la revendication 3, dans lequel le solvant utilisé à l'étape a) est du cyclohexane.

5. Procédé selon la revendication 2 ou la revendication 4, dans lequel l'additif ajouté à l'étape b) est choisi parmi le lactose, le mannitol, la polyvinylpyrrolidone, l'hydroxypropylméthylcellulose, la méthylcellulose, l'hydroxypropylcellulose, des agents gonflants tels que le carboxyméthylamide de sodium, la croscarmellose, la crospovidone, l'amidon prégélatinisé et les agents de modification du pH.

6. Procédé selon les revendications 2 à 5, dans lequel, à l'étape c), la séparation de phases a lieu sous l'effet d'une variation de la température.
